# FASCICULE DE BREVET EUROPEEN

(11) **EP 3 733 193 B1**
(45) Date de publication et mention de la délivrance du brevet: **20.12.2023**
(21) Numéro de dépôt: 20156120.6
(22) Date de dépôt: 07.02.2020
(51) Int. Cl.: A61K 33/00, A61P 29/00, A61K 9/00

(54) **MÉLANGE GAZEUX INHALABLE POUR TRAITER LES DOULEURS CHRONIQUES CHEZ DES PATIENTS RECEVANT DES MÉDICAMENTS DE PLUSIEURS CLASSES THÉRAPEUTIQUES**
INHALIERBARES GASGEMISCH ZUR BEHANDLUNG VON CHRONISCHEN SCHMERZEN BEI PATIENTEN, DIE ARZNEIMITTEL VERSCHIEDENER THERAPEUTISCHER KLASSEN ERHALTEN
INHALABLE GASEOUS MIXTURE FOR TREATING CHRONIC PAIN IN PATIENTS RECEIVING DRUGS FROM A PLURALITY OF THERAPEUTIC CLASSES

(30) Priorité: 02.05.2019 FR 1904626
(43) Date de publication de la demande: 04.11.2020
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: LECOURT, Laurent, 94250 Gentilly (FR); BESSIERE, Baptiste, 78350 Les Loges en Josas (FR); HOUETO, Patrick, 78350 Les Loges en Josas (FR); DELVAL, Cécile, 78350 Les Loges en Josas (FR); RAMIREZ, Juan Fernando, 78350 Les Loges en Josas (FR)
(74) Mandataire: Air Liquide

(56) Documents cités:
- WO-A1-2010/125271
- FR-A1- 2 981 276
- BOC Healthcare: "Entonox TM essential safety information", , 2018, XP002800228, Extrait de l'Internet: URL:Entonox%20Datasheet_tcm409-507933.pdf [extrait le 2020-09-07]

## Description

L'invention concerne un mélange gazeux contenant 50% de protoxyde d'azote (% mol.) et de l'oxygène, de préférence 50% d'oxygène (% mol.), utilisé en tant que médicament inhalable pour traiter thérapeutiquement des patients continuant à avoir des douleurs chroniques, en particulier des douleurs chroniques neuropathiques périphériques, et ce, malgré la prise d'au moins deux autres traitements antidouleurs différents, c'est-à-dire de substances ou composés médicamenteux relevant d'au moins deux classes thérapeutiques différentes, en particulier des patients réfractaires auxdites substances ou auxdits composés médicamenteux relevant d'au moins deux classes thérapeutiques différentes.

Les douleurs chroniques peuvent avoir un retentissement important sur la qualité de vie de nombreux patients, en particulier leur activité fonctionnelle, leur sommeil, leurs niveaux d'anxiété et de dépression... De ce fait, les patients souffrant de douleurs chroniques, en particulier de neuropathies périphériques, doivent être pris en charge thérapeutiquement, c'est-à-dire suivre des traitements médicamenteux de fond visant à diminuer ou à éliminer leurs douleurs.

A cette fin, il est recommandé de leur administrer d'abord des traitements médicamenteux de première intention, à savoir des antidépresseurs tricycliques, des antidépresseurs inhibiteurs de la recapture de la sérotonine et de la noradrénaline (e.g. duloxétine) ou encore des antiépileptiques agissant sur les canaux sodiques ou calciques (e.g. gabapentine, prégabaline...).

Toutefois, il a été constaté que ces médicaments ne sont pas efficaces chez certains patients puisque les douleurs persistent, malgré la prise de ces médicaments. Par ailleurs, d'autres patients ne tolèrent pas ces traitements car ces médicaments conduisent chez ces patients à des effets secondaires négatifs, tels que nausées, vomissements, somnolence, vertiges, détresses respiratoires... Or, ces effets secondaires sont difficilement supportables, i.e. mauvaise tolérance.

Il est alors nécessaire de recourir à d'autres médicaments, comme par exemple des opioïdes, ou des composés ou substances à visée plus locale, tel le qutenza.

Or, plus les patients sont traités et moins ils sont généralement répondeurs aux nouveaux traitements. De plus, ces patients sont également les plus fragilisés et dès lors supportent souvent mal les associations de différents traitements/médicaments, en particulier les associations de substances médicamenteuses ou composés médicamenteux relevant d'au moins deux classes thérapeutiques différentes, notamment des associations entre opioïdes, antidépresseurs, antiépileptiques et/ou d'autres anesthésiques locaux.

En d'autres termes, il est difficile de soulager certaines catégories particulières de patients souffrant de douleurs chroniques, en particulier de neuropathies périphériques, et qui sont traités par des associations de différents traitements, c'est-à-dire recevant des substances médicamenteuses ou des composés médicamenteux, d'au moins deux classes thérapeutiques différentes car ces patients supportent mal ces associations et/ou sont moins répondeurs à ces associations, en particulier des associations entre opioïdes, antidépresseurs, antiépileptiques et/ou d'autres anesthésiques locaux.

Par ailleurs, on connait FR-A-2981276 décrivant l'utilisation de xénon ou de N₂O pour traiter des douleurs neuropathiques particulières, à savoir celles induites par des sels de platine (i.e., cisplatine, oxaliplatine, carboplatine) ou une autre substance anticancéreuse chez une population particulière de patients, à savoir des patients atteints d'un cancer. Des teneurs allant jusqu'à 70% en volume de xénon ou de N₂O sont données mais il est précisé que les teneurs préférentielles sont de moins de 30% en volume.

On connait par ailleurs le document BOC Healthcare ; Entonox™ essential safety information ; 2018, qui enseigne un mélange gazeux contenant 50% de protoxyde d'azote (N₂O) et au moins 20% d'oxygène (O₂) (% molaires) conditionné en bouteille de gaz. Il permet de soulager la douleur lors d'une intervention de court terme de type soins dentaires, de suture de plaie, traitement de brûlures ou de traumatisme aigu, ou encore lors d'un accouchement normal.

En outre, FR-A-2944969 enseigne l'usage de N₂O pour traiter des douleurs neuropathiques chez un patient, en une teneur comprise entre 5 et 45% en volume, de préférence moins de 35% en volume.

Aucun de ces documents ne concerne la population particulière de patients susmentionnée, à savoir des patients recevant et/ou ayant déjà reçus au moins deux traitements (i.e. substances ou composés médicamenteux) de classes thérapeutiques différentes, c'est-à-dire de patients souffrant de douleurs chroniques, en particulier neuropathiques périphériques, et déjà traités par administration répétée d'au moins deux substances médicamenteuses de classes thérapeutiques différentes, que ces traitements aient été effectués par le passé et/ou soient toujours en cours, en particulier des associations entre opioïdes, antidépresseurs, antiépileptiques et/ou d'autres anesthésiques locaux.

Un problème est donc de pouvoir disposer d'un médicament amélioré permettant de soulager efficacement les patients souffrant de douleurs chroniques, en particulier de douleurs chroniques neuropathiques périphériques, et traités par ailleurs par administration d'au moins deux substances médicamenteuses de classes thérapeutiques différentes, c'est-à-dire une association médicamenteuse de plusieurs composés thérapeutiques, que ces substances médicamenteuses soient encore ou non administrées aux patients, de préférence avec une action du médicament sur plusieurs semaines et ce, hors de toute procédure d'anesthésie.

En d'autres termes, il existe un besoin médical persistant dans la prise en charge des douleurs chroniques, en particulier de douleurs chroniques neuropathiques périphériques, notamment par des traitements plus efficaces et mieux tolérés par les patients que les stratégies actuelles, en particulier dans la population de patients déjà traités par au moins deux substances médicamenteuses (i.e. composé ou produit) de classes thérapeutiques différentes, en particulier les patients réfractaires aux traitements par lesdites au moins deux substances médicamenteuses de classes thérapeutiques différentes, et ce, notamment en vue d'améliorer le rapport bénéfice/risque de tels traitements.

Dit encore autrement, il est souhaitable de pouvoir améliorer l'efficacité et la tolérance de patients souffrants de douleur chroniques, en particulier de douleurs chroniques neuropathiques périphériques, et traités par au moins deux substances (i.e. composé ou produit) médicamenteuses de classes thérapeutiques différentes utilisées pour le traitement de la douleur chronique, lesquels patients sont réfractaires auxdites au moins deux substances médicamenteuses de classes thérapeutiques différentes.

Une solution selon l'invention concerne un mélange gazeux contenant 50% de protoxyde d'azote (N₂O) et au moins 20% d'oxygène (O₂) (% molaires) pour une utilisation en tant que médicament inhalable pour traiter des douleurs chroniques chez un patient humain traité par plusieurs substances médicamenteuses d'au moins deux classes thérapeutiques différentes, de préférence un patient réfractaire auxdites substances médicamenteuses d'au moins deux classes thérapeutiques différentes, lesdites substances médicamenteuses étant choisies parmi les opioïdes, les antiépileptiques (AE), les antidépresseurs (AD) et les anesthésiques locaux.

En d'autres termes, le mélange gazeux contenant 50% de protoxyde d'azote (N₂O) et au moins 20% d'oxygène (O₂) (% mol.) est utilisé dans le cadre de l'invention en tant que médicament inhalable pour traiter des douleurs chroniques chez un patient humain ayant déjà été traité par plusieurs type de composés médicamenteux relevant de classes thérapeutiques différentes ayant également une action contre la douleur chronique mais ayant montré une faible ou non-efficacité et/ou ayant conduit à une intolérance chez le patient considéré, c'est-à-dire un patient réfractaire à ces traitements.

De plus, de manière surprenante, lorsqu'un mélange gazeux contenant 50% de N₂O et au moins 20% d'oxygène O₂, notamment le MEOPA, selon l'invention a été administré chez à des patients traités préalablement par plusieurs types de composés médicamenteux relevant de classes thérapeutiques différentes, les résultats en termes d'efficacité et de tolérance sont meilleurs que ceux obtenus sur des patients traités par seulement un de ces composés médicamenteux.

Dans le cadre de la présente invention :
- un patient réfractaire est une personne chez laquelle un traitement à base des composés médicamenteux relevant de classes thérapeutiques différentes, qui lui a été administré préalablement pendant une durée longue, typiquement plusieurs jours, semaines ou mois, pour tenter de soulager ses douleurs chroniques, n'est pas parvenu à produire un effet suffisant pour diminuer lesdites douleurs chroniques, donc chez lequel les composés médicamenteux relevant de classes thérapeutiques différentes ne sont pas ou peu efficaces et/ou mal tolérés, notamment du fait d'effets secondaires possibles liés à une posologie incompatible avec le patient considéré.
- les substances médicamenteuses sont administrées en traitement de fond de la douleur, en particulier pendant des durées longues, typiquement pendant plusieurs jours, semaines ou mois, de préférence plusieurs mois, voire années, avec une ou des administrations journalières de ces composés médicamenteux ; toutefois, ce traitement de fond par substances médicamenteuses relevant de classes thérapeutiques différentes ne fait pas partie de la présente invention.
- les proportions de composés (N₂O, O₂...) dans le mélange gazeux sont exprimées en pourcentages molaires (% mol.).
- les termes « substance médicamenteuse », « produit médicamenteux » et « composé médicamenteux » sont considérés comme totalement équivalents et sont utilisés indifféremment.

Selon le mode de réalisation considéré, le mélange gazeux utilisé en tant que médicament inhalable selon l'invention peut comprendre l'une ou plusieurs des caractéristiques suivantes :
- il contient au moins 30% d'oxygène, de préférence au moins 40% d'oxygène.
- il contient au maximum 50% d'oxygène environ.
- il contient de l'oxygène, du N₂O et de l'azote (N₂).
- il est constitué de 50% de protoxyde d'azote (N₂O) et 50% d'oxygène (O₂). Ce mélange équimolaire (50%/50%) est aussi appelé « MEOPA » pour Mélange Equimolaire d'Oxygène et de Protoxyde d'Azote.
- bien entendu, même si cela n'est ni souhaitable, ni voulu, le mélange gazeux N₂O/O₂ selon l'invention, tel du MEOPA, peut contenir éventuellement des impuretés inévitables, par exemple des impuretés de type CO, CO₂, H₂O et NOₓ. Toutefois, on veille à ce que le niveau d'impureté soit aussi faible de possible, notamment inférieur à 1% environ, typiquement inférieur à 500 ppm, par exemple moins de 300 ppm pour les impuretés de type CO et CO₂, moins de 5 ppm pour les impuretés de type H₂O et moins de 2 ppm pour les impuretés de type NOₓ.
- le protoxyde d'azote est le principe actif.
- le patient est un être humain, à savoir un homme ou une femme, que ce soit un enfant, un adolescent, un adulte, y compris les personnes âgées.
- le patient ne répond pas ou mal aux substances médicamenteuses d'au moins deux classes thérapeutiques différentes lui ayant été administrées.
- le patient est réfractaire aux substances médicamenteuses d'au moins deux classes thérapeutiques différentes lui ayant été administrées.
- le patient humain est douloureux chronique.
- le patient a développé une intolérance à au moins l'une des substances médicamenteuses d'au moins deux classes thérapeutiques différentes lui ayant été administrées.
- le patient reçoit le mélange gazeux de protoxyde d'azote et d'oxygène de manière concomitante, i.e. en même temps, que les substances médicamenteuses d'au moins deux classes thérapeutiques différentes.
- le patient traité reçoit le mélange gazeux de protoxyde d'azote et d'oxygène subséquemment, i.e. après, aux substances médicamenteuses d'au moins deux classes thérapeutiques différentes. Toutefois, cela n'impose pas que les substances médicamenteuses soient prises obligatoirement en association durant toute la durée du traitement par le mélange gazeux de protoxyde d'azote et d'oxygène
- les substances médicamenteuses d'au moins deux classes thérapeutiques différentes sont choisies parmi les opioïdes, les antiépileptiques (AE) et les antidépresseurs (AD).
- les substances médicamenteuses d'au moins deux classes thérapeutiques différentes comprennent un composé antiépileptique choisi parmi gabapentine et prégabaline, ou tout autre composé AE à mécanisme d'action identique ou analogue.
- les substances médicamenteuses d'au moins deux classes thérapeutiques différentes comprennent un composé antidépresseur :
   o de type tricyclique, de préférence nortriptyline, desipramine ou amitriptyline, ou
   ∘ inhibiteur de la recapture serotonine-norepinephrine, de préférence duloxetine.
- les substances médicamenteuses d'au moins deux classes thérapeutiques différentes comprennent un composé opioïde choisi parmi tramadol, codéine, oxycodone, tapentadole, fentanyl, remifentanyl et morphine, c'est-à-dire la morphine elle-même ou tout autre dérivé naturel ou chimique de la morphine.
- les substances médicamenteuses d'au moins deux classes thérapeutiques différentes comprennent un composé anesthésique local choisi parmi lidocaïne et capsaïcine.
- les douleurs chroniques sont des douleurs neuropathiques.
- les douleurs chroniques présentent au moins une composante périphérique, par exemple une douleur mixte.
- les douleurs chroniques sont des douleurs neuropathiques périphériques.
- les douleurs chroniques sont des douleurs neuropathiques périphériques liées à et/ou résultant d'une (ou des) lésion ou irritation d'un ou plusieurs nerfs périphériques.
- la lésion ou irritation du ou des nerfs périphériques est d'origine traumatique, toxique, métabolique, ischémique, immuno-allergique ou infectieuse.
- les douleurs neuropathiques chroniques périphériques résultent ou sont liées à une lésion ou irritation d'un ou plusieurs nerfs périphériques.
- les douleurs neuropathiques chroniques périphériques sont choisies notamment parmi les douleurs post-zostériennes, les douleurs des neuropathies diabétiques, les polyneuropathies, et les douleurs post-traumatiques ou post-opératoires.
- les douleurs sont spontanées et/ou provoquées.
- les douleurs spontanées sont continues et/ou superficielles (e.g. sensation de brûlures) ou profondes (e.g. sensation d'un serrage par un étau ou de compression), avec ou sans survenue d'accès paroxystiques (i.e. sensation de décharge électrique) et de paresthésies/dysesthésies.
- les douleurs provoquées se caractérisent par une hypersensibilité au chaud, au froid ou au toucher.
- le mélange gazeux n'est pas administré pendant une anesthésie, c'est-à-dire qu'il est destiné à des patients conscients, i.e. non-anesthésiés. Dit autrement, le mélange gazeux selon l'invention n'est pas destiné à être utilisé dans le cadre d'une procédure d'anesthésie chez des patients devant subir une telle procédure d'anesthésie.
- le mélange gazeux est conditionné dans un récipient de gaz, en particulier une bouteille de gaz.
- alternativement, le mélange gazeux peut aussi être obtenu ou fabriqué par mélange extemporané des composés (N₂O, O₂) sur le site d'utilisation au moyen d'un mélangeur de gaz ou analogue.

Le mélange gazeux permet de soulager un patient souffrant d'une ou de douleurs chroniques, en particulier de douleurs neuropathiques chroniques périphériques (DNCP), et ayant besoin d'un traitement complémentaire pour soulager au moins partiellement ses douleurs chroniques, comme suit:
a) on sélectionne un patient recevant et/ou ayant reçu, pour tenter de traiter ces douleurs chroniques, plusieurs substances médicamenteuses d'au moins deux classes thérapeutiques différentes, c'est-à-dire un patient dans un stade plus avancé de la maladie, de préférence des patients réfractaires auxdites substances médicamenteuses d'au moins deux classes thérapeutiques différentes, et
b) on administre par inhalation audit patient un mélange gazeux N₂O/O₂ contenant 50% de protoxyde d'azote (N₂O) et au moins 20% d'oxygène (O₂) (% mol.) selon l'invention, de préférence un mélange équimolaire 50%/50% N₂O/O₂.

Selon le mode de réalisation considéré, l'invention peut comprendre l'une ou plusieurs des caractéristiques suivantes :
- le mélange gazeux est administré au moins une fois par jour.
- le mélange gazeux est administré au moins une fois par jour pendant 1 à plusieurs jours, de préférence plusieurs jours consécutifs.
- le mélange gazeux est administré pendant une durée de 1 à 3 jours.
- de préférence, on répète l'administration à des intervalles de temps d'au moins 1 semaine, de préférence d'au moins 2 à 4 semaines, en fonction de la réponse au traitement.
- le mélange gazeux est administré pendant au moins 30 min de préférence au moins 45 min avantageusement au moins 1 heure à chaque administration typiquement entre 55 min et 65 min, c'est à dire pendant une durée suffisant pour obtenir un effet long-terme sur plusieurs semaines
- le mélange gazeux est administré à un débit compris entre 1 et 25 L/min, de préférence entre 2 et 16 L/min.
- le patient est un adulte ou un enfant (i.e. > 2 ans).
- le mélange gazeux est administré pendant une durée longue, en particulier une ou plusieurs années.

Un système d'administration du mélange gazeux N_{2/}O₂ peut comprendre
- un récipient de gaz contenant le mélange gazeux N₂O/O₂ à administrer,
- une interface respiratoire de fourniture de gaz au patient.

Selon le mode de réalisation considéré, le système d'administration selon l'invention peut comprendre l'une ou plusieurs des caractéristiques suivantes :
- le récipient de gaz est équipé d'un bloc robinet de distribution de gaz, de préférence un bloc robinet à détendeur intégré ou RDI.
- le récipient de gaz a un corps cylindrique.
- le récipient de gaz a un corps cylindrique en métal ou en matériaux composites, notamment en alliage d'aluminium ou en acier.
- le récipient de gaz est équipé d'un bloc robinet de distribution de gaz protégé par un capotage de protection, aussi appelé « chapeau », en polymère ou en métal.
- l'interface respiratoire de fourniture de gaz au patient est un masque.....
- l'interface respiratoire est reliée fluidiquement au récipient de gaz par un tuyau flexible ou analogue servant à véhiculer le gaz.
- le récipient de gaz contient un mélange gazeux N₂O/O₂ comprenant 50% de protoxyde d'azote (N₂O) et au moins 20% d'oxygène (O₂) (% mol.), de préférence au moins 30% d'oxygène (O₂), typiquement un mélange équimolaire 50%/50% N₂O/O₂.

Selon une autre variante de réalisation, le mélange gazeux contenant 50% de protoxyde d'azote (N₂O) et au moins 20% d'oxygène (O₂) (% molaires) selon l'invention pour une utilisation en tant que médicament inhalable pour traiter des douleurs chroniques chez un patient humain traité par plusieurs substances médicamenteuses d'au moins deux classes thérapeutiques différentes, est obtenu ou fabriqué par mélange extemporané des composés (O₂, N₂O) sur le site d'utilisation au moyen d'un mélangeur de gaz ou analogue.

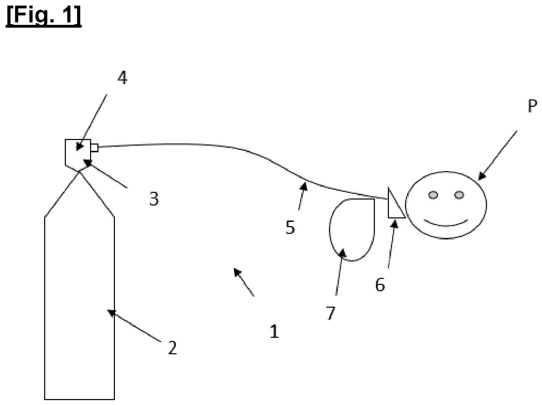

L'invention va maintenant être mieux comprise grâce à la description détaillée suivante, faite à titre illustratif mais non limitatif, à la Figure 1 annexée et aux exemples comparatifs consignés ci-après, où la Figure 1 schématise un système d'administration du mélange gazeux.

La Figure 1 schématise un mode de réalisation d'un système d'administration 1 d'un mélange gazeux N₂O/O₂ à un patient humain souffrant de douleurs chroniques, en particulier neuropathiques, et traité par ailleurs par plusieurs substances médicamenteuses, i.e. au moins deux, d'au moins deux classes thérapeutiques différentes qui lui sont administrées dans le but de soulager ses douleurs chroniques mais lesquelles ne présentent pas ou peu d'efficacité et/ou sont susceptibles d'être mal tolérées par le patient.

De préférence, le mélange gazeux est constitué de 50% de protoxyde d'azote (N₂O) et 50% d'oxygène (O₂), et éventuellement des impuretés inévitables résultant par exemple du procédé de fabrication du mélange gazeux. Ce mélange gazeux binaire équimolaire N₂O/O₂ (50%/50%) est appelé MEOPA pour Mélange Equimolaire Oxygène Protoxyde d'Azote.

Ce système d'administration 1 comprend une bouteille de gaz 2 équipée d'un robinet 3 à détendeur intégré (RDI) contenant le mélange gazeux N₂O/O₂, tel le MEOPA, qui y est conditionné à une pression pouvant atteindre 300 bar abs, appelée « pression haute ». Le RDI 3 permet de réduire la pression du mélange gazeux N₂O/O₂ jusqu'à une pression plus basse, appelée « pression basse » ou « pression de sortie », par exemple de 5 bar abs ou moins, qui est délivré par un raccord 4 de sortie du RDI 3.

Le mélange N₂O/O₂ est acheminé jusqu'au patient P, via un tuyau flexible 5, qui est en communication fluidique, d'une part, avec le raccord de sortie 4 du RDI 3 et, d'autre part, avec une interface 6 de distribution du gaz, tel un masque respiratoire.

Préférentiellement, un réservoir-tampon 7, tel un ballon-flexible, peut être inséré en amont de l'interface respiratoire 6 pour faciliter l'administration du gaz au patient P.

Bien entendu on pourra utiliser d'autre dispositif d'administration adapté à la fourniture de ce mélange au patient.

Le mélange gazeux N₂O/O₂ est un médicament inhalable servant à traiter les douleurs chroniques, en particulier des douleurs chroniques neuropathiques, préférentiellement d'origine périphérique, chez le patient P qui a par ailleurs déjà reçu plusieurs substances médicamenteuses, i.e. au moins deux, d'au moins deux classes thérapeutiques différentes (i.e. ces traitements préalables peuvent arrêtes ou toujours en cours), lesquelles substances médicamenteuses n'ont pas permis ou ne permettent pas de faire disparaître les douleurs du patient, du fait par exemple d'un manque d'efficacité, en particulier un patient réfractaire auxdites substances médicamenteuses.

Ces substances médicamenteuses peuvent être par exemple un ou des antiépileptiques (AE) de type prégabaline ou gabapentine ou autres, un ou des antidépresseurs (AD) de type fluoxétine ou autres, ou un ou des opioïdes, ou un ou des anesthésiques locaux.

D'une façon générale, les douleurs chroniques neuropathiques se distinguent par leur physiopathologie et leur expression clinique. Elles ont été définies par l'International *Association for the Study of Pain,* comme des douleurs secondaires à une lésion ou à une maladie affectant le système somato-sensoriel. Elles peuvent être d'origine périphérique ou d'origine centrale.

Dans le cadre de la présente invention, on considère préférentiellement les douleurs chroniques neuropathiques ayant une composante périphérique, aussi appelées douleurs neuropathiques chroniques périphériques (DNCP). Celles-ci surviennent par exemple à la suite d'une lésion ou irritation d'origine traumatique, toxique, métabolique, ischémique, immuno-allergique ou infectieuse des nerfs périphériques.

Elles sont choisies notamment parmi les douleurs post-zostériennes, les douleurs des neuropathies diabétiques, les polyneuropathies, et les douleurs post-traumatiques ou post-opératoires.

Elles se caractérisent par des douleurs spontanées et/ou provoquées.

Les douleurs spontanées peuvent être continues et/ou superficielles (e.g. sensation de brûlures) ou profondes (e.g. sensation d'un serrage par un étau ou de compression) et peuvent engendrer une survenue d'accès paroxystiques (i.e. sensation de décharge électrique) et de paresthésies/dysesthésies. Les douleurs provoquées se caractérisent quant à elles par des hypersensibilités au chaud au froid, au toucher et sont aussi appelées « hyperalgies » ou « allodynies ».

Afin de montrer l'efficacité du médicament inhalable N₂O/O₂ selon l'invention, les exemples comparatifs suivants ont été réalisés.

### Exemples

Des essais comparatifs ont été réalisés dans 2 pays et dans 22 hôpitaux, en utilisant du MEOPA (50/50% N₂O/O₂) selon l'invention et de l'air médical servant de témoin (i.e. gaz placebo).

Ces mélanges gazeux ont été administrés par inhalation à des patients adultes souffrant de douleurs neuropathiques chroniques périphériques (DNCP) et déjà traités (i.e. traitements en cours ou stoppés) par une ou plusieurs substances médicamenteuses, en particulier au moins deux substances médicamenteuses d'au moins deux classes thérapeutiques différentes ou, à titre comparatif, des patients non traités, en utilisant un même système d'administration tel que décrit en [Fig. 1].

Les substances médicamenteuses d'au moins deux classes thérapeutiques différentes sont un ou des opioïdes, antiépileptiques (AE), antidépresseurs (AD) et anesthésiques locaux.

Les patients ont été tirés au sort pour recevoir soit le MEOPA, soit de l'air médical. Toutefois, les patients ne sont pas informés du gaz qu'ils inhalent. Les patients reçoivent les gaz testés alors qu'ils sont éveillés et conscients (i.e. hors toute anesthésie).

Les traitements ont été administrés pendant 1 heure par jour pendant 3 jours consécutifs. Chaque patient de l'étude était suivi pendant 4 semaines et devait compléter tout au long du suivi plusieurs questionnaires (i.e. NRS, SF-12, NPSI, PGIC) permettant d'évaluer l'évolution :
- de l'intensité de la douleur. Le questionnaire NRS permet aux patients d'évaluer l'intensité de leur douleur sur une échelle allant de 0 (absence de douleur) à 10 (douleur la plus forte possible).
- de la qualité de vie. Le questionnaire SF-12 représente une échelle de qualité vie. Elle comporte 8 dimensions permettant d'évaluer sur les 4 semaines précédant l'administration, la composante physique et la composante mentale de la qualité de vie de chaque patient.
- des différents composantes de la douleur neuropathique. Le questionnaire NPSI permettant de caractériser les différentes composantes de la douleur. Il comprend 12 questions donnant un score total et 5 sous-scores représentant les dimensions suivantes: douleurs spontanées superficielles à type de brûlures, douleurs spontanées profondes à type de compression, d'étau, douleurs paroxysmales à type de décharges électriques, de coups de couteau, paresthésies et dysesthésies à type de fourmillements, douleurs provoquées par les frottements, et contact du froid.
- du ressenti douloureux global. Le questionnaire PGIC est une échelle qui permet de mesurer l'évolution sous traitement des limitations d'activité, des symptômes, des émotions et de la qualité de vie globale qui sont liés à la condition douloureuse. Cette évolution est mesurée en 7 points allant de "très détériorée" à "très améliorée".

Les résultats des essais sont obtenus sur des patients ayant reçu trois administrations, soit de MEOPA, soit de placebo (i.e. air) dont au moins une administration complète (i.e. durée d'administration allant de 55 à 65 minutes avec des pauses cumulées n'excédant pas 5 minutes) et ayant fait au moins 4 évaluations de l'intensité de leur douleur durant la première semaine après le traitement.

Les résultats sont consignés dans les Tableaux ci-après où :
[Tableau 1] donne les résultats de l'évolution de l'intensité de la douleur (score NRS) après 1 semaine de suivi sur la population globale des patients DNCP de l'étude, et
[Tableau 2] consigne les résultats de l'évolution de l'intensité de la douleur (score NRS) sur les 4 semaines de suivi chez les patients DNCP de l'étude.

**[Tableau 1]**

| Nb de substances médicamenteuses reçues | Patients recevant du MEOPA (103 patients) | Patients recevant un placebo (air) (118) | Significativité (p) |
|---|---|---|---|
| 0 | 21 patients | 28 patients | |
| | -0.93 (1.4) | -0.98 (1.5) | p=0.79 |
| 1 | 36 patients | 33 patients | Non significatif |
| | -0.76 (1.6) | -0.86 (1.1) | |
| 2 | 33 patients | 42 patients | |
| | -1.08 (1.2) | -0.54 (1.1) | p=0.04 |
| 3 | 13 patients | 15 patients | Significatif |
| | -1.74 (1.8) | -1.13 (1.7) | |

Dans chaque cellule du [Tableau 1], sont donnes : le nombre de patients concernés, différence du score NRS entre la 1ère semaine suivant le traitement et la semaine avant le traitement, et écart-type de cette différence.

On constate, de manière surprenante, au vu des résultats du [Tableau 1], que les patients qui ont été traités par au moins deux substances médicamenteuses servant à soulager leur douleur chronique (i.e. opioïdes, AE, AD et/ou anesthésiques locaux) ont un niveau de réponse au MEOPA supérieur à celui des patients qui ont été traités par uniquement une seule substance médicamenteuse ou à celui de ceux n'ayant reçu aucun traitement préalable de leur douleur chronique.

En d'autres termes, les résultats obtenus montrent une différence statistiquement significative en faveur du MEOPA concernant la réduction de l'intensité de la douleur pour les patients traités par MEOPA et ayant reçus deux ou trois autres substances médicamenteuses servant à soulager leur douleur chronique comparé aux autres patients, c'est-à-dire ceux traités soit par de l'air médical (placebo) quel que soit le nombre de substances médicamenteuses leur ayant été administrées par ailleurs, soit du MEOPA et au maximum une autre substance médicamenteuse (i.e. 1 ou 0).

Ceci est étonnant car, en général, le niveau de réponse des patients naïfs ou peu traités est d'habitude meilleur que celui des patients lourdement traités. Or, ici, c'est l'inverse.

Ceci démontre l'efficacité de l'administration par inhalation d'un mélange gazeux selon l'invention contenant 50% de protoxyde d'azote (N₂O) et au moins 20% d'oxygène (O₂) (% molaires) aux patients traités par ailleurs par plusieurs substances médicamenteuses, i.e. au moins deux substances médicamenteuses, lesquelles relèvent de classes thérapeutiques différentes, à savoir AE, AD, opioïdes ou anesthésiques locaux, afin de traiter leurs douleurs chroniques, en particulier des douleurs neuropathiques périphériques.

**[Tableau 2]**

| Nombre de semaines après administration de MEOPA | Patients recevant au moins 2 substances médicamenteuses et répondeurs à 30% (*) (46 patients) | | Patients recevant 0 ou 1 substance médicamenteuse et répondeurs à 30%(*) (57 patients) | |
|---|---|---|---|---|
| 1 | 13/46 | (28.3%) | 15/57 | (26.3%) |
| 2 | 15/46 | (32.6%) | 11/55 | (20.0%) (**) |
| 3 | 16/46 | (35.6%) | 14/55 | (25.5%) (**) |
| 4 | 13/46 | (28.9%) | 12/54 | (22.2%) (**) |

| | | | | |
|---|---|---|---|---|
| (*) : Nombre de patients répondeurs à 30%, c'est-à-dire pour lesquels on constate une baisse de 30% de leur score NRS. (**) : les questionnaires de 2 ou 3 patients n'étaient pas évaluables ou pas exploitables. | | | | |

Le [Tableau 2] permet de comparer l'efficacité du MEOPA chez des patients traités par de 0 à 3 autres substances médicamenteuses de type AE, AD, opioïdes ou anesthésiques locaux, plus particulièrement chez les patients présentant une baisse de 30% de leur score NRS.

Les résultats obtenus montrent clairement une proportion (%) supérieure de patients attestant d'une réduction de l'intensité de leur douleur chronique de la semaine 1 à la semaine 4, dans le groupe de patients selon l'invention, c'est-à-dire qui ont reçu par ailleurs au moins 2 substances médicamenteuses relevant de classes thérapeutiques différentes (i.e. AE, AD, opioïdes, anesthésiques locaux).

Cette différence apparaît dès le départ, devient plus significative à partir de la deuxième semaine et se poursuit pendant 4 semaines.

Ces résultats sont surprenants pour plusieurs raisons.

Tout d'abord, ils confirment que le médicament contenant 50% de N₂O a une efficacité ciblée dans la lutte contre la douleur sur une (sous-)population de patients particulière, à savoir les patients traités par plusieurs substances médicamenteuses (i.e. AE, AD, opioïdes, anesthésiques locaux), alors que son efficacité est moindre sur ceux traités par une seule substance médicamenteuse ou aucune.

Ensuite, ils démontrent une persistance de l'efficacité du médicament inhalable ici du MEOPA, pendant une durée de plusieurs semaines, à savoir ici 4 semaines, chez ces mêmes patients après une administration courte. Ce deuxième effet surprenant n'existe avec aucun des autres traitements de fond à administration générale reçus par les patients.

Enfin, il est à souligner qu'aucun de ces patients n'a montré de réaction secondaire au MEOPA, c'est-à-dire que la tolérance du MEOPA est satisfaisante sur dans cette (sous)population de patients, i.e. les patients traités par plusieurs substances médicamenteuses de classes différentes (i.e. AE, AD, opioïdes, anesthésiques locaux).

## Revendications

1. Mélange gazeux contenant 50% de protoxyde d'azote (N₂O) et au moins 20% d'oxygène (O₂) (% molaires) pour une utilisation en tant que médicament inhalable pour traiter des douleurs chroniques chez un patient humain traité par plusieurs substances médicamenteuses d'au moins deux classes thérapeutiques différentes, lesdites substances médicamenteuses étant choisies parmi les opioïdes, les antiépileptiques (AE), les antidépresseurs (AD) et les anesthésiques locaux.

2. Mélange gazeux pour une utilisation selon la revendication 1, **caractérisé en ce que** le patient est réfractaire aux substances médicamenteuses d'au moins deux classes thérapeutiques différentes.

3. Mélange gazeux pour une utilisation selon la revendication 1, **caractérisé en ce qu'**il contient au moins 30% d'oxygène, de préférence au moins 40% d'oxygène (% molaires).

4. Mélange gazeux pour une utilisation selon l'une des revendications 1 ou 3, **caractérisé en ce qu'**il contient de l'oxygène, du N₂O et de l'azote (N₂).

5. Mélange gazeux pour une utilisation selon l'une des revendications 1 ou 3, **caractérisé en ce qu'**il est constitué de 50% de protoxyde d'azote (N₂O) et 50% d'oxygène (O₂) (% molaires).

6. Mélange gazeux pour une utilisation selon la revendication 1, **caractérisé en ce que** les substances médicamenteuses d'au moins deux classes thérapeutiques différentes comprennent :
- un composé antiépileptique choisi parmi gabapentin et pregabaline,
- un composé antidépresseur :
o de type tricyclique, de préférence nortriptyline, desipramine ou amitriptyline, ou
o inhibiteur de la recapture serotonine-norepinephrine, de préférence duloxetine,
- un composé opioïde choisi parmi tramadol, codéine, oxycodone, tapentadole, fentanyl, remifentanyl et morphine, et/ou
- un composé anesthésique local choisi parmi lidocaïne et capsaïcine.

7. Mélange gazeux pour une utilisation selon la revendication 1, **caractérisé en ce que** les douleurs chroniques sont des douleurs neuropathiques.

8. Mélange gazeux pour une utilisation selon l'une des revendications 1 ou 7, **caractérisé en ce que** les douleurs chroniques sont des douleurs neuropathiques périphériques.

9. Mélange gazeux pour une utilisation selon l'une des revendications 1, 7 ou 8, **caractérisé en ce que** les douleurs neuropathiques chroniques périphériques résultent ou sont liées à une lésion ou irritation d'un ou plusieurs nerfs périphériques.

10. Mélange gazeux pour une utilisation selon la revendication 1, **caractérisé en ce que** le patient est un adulte, un adolescent ou un enfant.

11. Mélange gazeux pour une utilisation selon l'une des revendications 1 à 10, **caractérisé en ce qu'**il est formulé pour une administration d'au moins une fois par mois et/ou d'au moins une fois par jour pendant une durée d'administration d'au moins 30 min.

12. Mélange gazeux pour une utilisation selon l'une des revendications 1 à 11, **caractérisé en ce qu'**il formulé pour une administration à un débit compris entre 1 et 25 L/min.

## Patentansprüche

1. Gasgemisch, enthaltend 50 % Lachgas (N₂O) und mindestens 20 % Sauerstoff (O₂) (Mol-%) zur Verwendung als inhalierbares Arzneimittel zur Behandlung chronischer Schmerzen bei einem durch mehrere Arzneimittelsubstanzen aus mindestens zwei verschiedenen Therapieklassen behandelten menschlichen Patienten, wobei die Arzneimittelsubstanzen aus Opioiden, Antiepileptika (AE), Antidepressiva (AD) und Lokalanästhetika ausgewählt sind.

2. Gasgemisch zur Verwendung nach Anspruch 1, **dadurch gekennzeichnet, dass** der Patient gegenüber den Arzneimittelsubstanzen aus mindestens zwei verschiedenen Therapieklassen refraktär ist.

3. Gasgemisch zur Verwendung nach Anspruch 1, **dadurch gekennzeichnet, dass** es mindestens 30 % Sauerstoff, vorzugsweise mindestens 40 % Sauerstoff (Mol-%) enthält.

4. Gasgemisch zur Verwendung nach einem der Ansprüche 1 oder 3, **dadurch gekennzeichnet, dass** es Sauerstoff, N₂O und Stickstoff (N₂) enthält.

5. Gasgemisch zur Verwendung nach einem der Ansprüche 1 oder 3, **dadurch gekennzeichnet, dass** es zu 50 % aus Lachgas (N₂O) und zu 50 % aus Sauerstoff (O₂) (Mol-%) besteht.

6. Gasgemisch zur Verwendung nach Anspruch 1, **dadurch gekennzeichnet, dass** die Arzneimittelsubstanzen aus mindestens zwei verschiedenen Therapieklassen:
- eine aus Gabapentin und Pregabalin ausgewählte Antiepileptikum-Verbindung,
- eine Antidepressivum-Verbindung:
o des trizyklischen Typs, vorzugsweise Nortriptylin, Desipramin oder Amitriptylin, oder
o des Serotonin-Noradrenalin-Wiederaufnahmehemmer-Typs, vorzugsweise Duloxetin,
- eine aus Tramadol, Codein, Oxycodon, Tapentadol, Fentanyl, Remifentanyl und Morphin ausgewählte Opioidverbindung und/oder
- eine aus Lidocain und Capsaicin ausgewählte Lokalanästhetikum-Verbindung
umfassen.

7. Gasgemisch zur Verwendung nach Anspruch 1, **dadurch gekennzeichnet, dass** es sich bei den chronischen Schmerzen um neuropathische Schmerzen handelt.

8. Gasgemisch zur Verwendung nach einem der Ansprüche 1 oder 7, **dadurch gekennzeichnet, dass** es sich bei den chronischen Schmerzen um periphere neuropathische Schmerzen handelt.

9. Gasgemisch zur Verwendung nach einem der Ansprüche 1, 7 oder 8, **dadurch gekennzeichnet, dass** die chronischen peripheren neuropathischen Schmerzen aus einer Läsion oder Reizung eines oder mehrerer peripherer Nerven resultieren oder damit in Zusammenhang stehen.

10. Gasgemisch zur Verwendung nach Anspruch 1, **dadurch gekennzeichnet, dass** der Patient ein Erwachsener, ein Jugendlicher oder ein Kind ist.

11. Gasgemisch zur Verwendung nach einem der Ansprüche 1 bis 10, **dadurch gekennzeichnet, dass** es zur mindestens einmal monatlichen und/oder mindestens einmal täglichen Verabreichung für eine Verabreichungsdauer von mindestens 30 min formuliert ist.

12. Gasgemisch zur Verwendung nach einem der Ansprüche 1 bis 11, **dadurch gekennzeichnet, dass** es zur Verabreichung bei einer Durchflussmenge zwischen 1 und 25 l/min formuliert ist.

## Claims

1. Gas mixture containing 50% of nitrous oxide (N₂O) and at least 20% of oxygen (O₂) (mol%) for use as an inhalable drug for treating chronic pain in a human patient treated with several drug substances from at least two different therapeutic classes, said drug substances being chosen from opioids, antiepileptics (AEs), antidepressants (ADs) and local anaesthetics.

2. Gas mixture for use according to Claim 1, **characterized in that** the patient is refractory to drug substances from at least two different therapeutic classes.

3. Gas mixture for use according to Claim 1, **characterized in that** it contains at least 30% of oxygen, preferably at least 40% of oxygen (mol%).

4. Gas mixture for use according to either of Claims 1 and 3, **characterized in that** it contains oxygen, N₂O and nitrogen (N₂).

5. Gas mixture for use according to either of Claims 1 and 3, **characterized in that** it consists of 50% of nitrous oxide (N₂O) and 50% of oxygen (O₂) (mol%) .

6. Gas mixture for use according to Claim 1, **characterized in that** the drug substances from at least two different therapeutic classes comprise:
- an antiepileptic compound selected from gabapentin and pregabalin,
- an antidepressant compound:
○ of the tricyclic type, preferably nortriptyline, desipramine or amitriptyline, or
○ of the serotonin-norepinephrine reuptake inhibitor type, preferably duloxetine,
- an opioid compound selected from tramadol, codeine, oxycodone, tapentadole, fentanyl, remifentanyl and morphine, and/or
- a local anaesthetic compound selected from lidocaine and capsaicin.

7. Gas mixture for use according to Claim 1, **characterized in that** the chronic pain is neuropathic pain.

8. Gas mixture for use according to either of Claims 1 and 7, **characterized in that** the chronic pain is peripheral neuropathic pain.

9. Gas mixture for use according to one of Claims 1, 7 and 8, **characterized in that** the chronic peripheral neuropathic pain results from or is associated with a lesion or irritation of one or more peripheral nerves.

10. Gas mixture for use according to Claim 1, **characterized in that** the patient is an adult, a teenager or a child.

11. Gas mixture for use according to one of Claims 1 to 10, **characterized in that** it is formulated for administration at least once a month and/or at least once a day for a period of administration of at least 30 min.

12. Gas mixture for use according to one of Claims 1 to 11, **characterized in that** it is formulated for administration at a flow rate of between 1 and 25 l/min.
